# EUROPEAN PATENT APPLICATION

(11) **EP 4 438 105 A1**
(43) Date of publication of application: **02.10.2024**
(21) Application number: 22899042.0
(22) Date of filing: 23.11.2022
(51) Int. Cl.: A61N 1/36, A61N 1/04, A61M 21/00

(54) **METHOD FOR CONTROLLING COMPLEX STIMULATION AND COMPLEX STIMULATION DEVICE**

(30) Priority: 26.11.2021 KR 20210165569
(71) Applicant: Neurive Co., Ltd., Juchon-myeon Gimhae-si, Gyeongsangnam-do 50969 (KR); Korea University Research and Business Foundation, Seoul 02841 (KR)
(72) Inventor: SONG, Jae Jun, Seoul 06501 (KR); CHOI, Hyuk, Seoul 06095 (KR); HONG, Ki Hwan, Seoul 03709 (KR)
(74) Representative: Franke, Dirk
(86) International application number: PCT/KR2022/018654
(87) International publication number: WO 2023/096360

(57) **Abstract**

The present invention relates to a method for controlling a complex stimulation, which is a method for controlling a complex stimulation device having an electrical stimulation unit including electrodes in contact with human ears and a sound output unit for stimulating hearing by using sound waves, wherein the sound output unit generates sound waves having different frequencies in both ears, and controls the frequency difference between the sound waves applied to both ears on the basis of frequencies of electrical stimulation applied to the electrodes. The present invention has an excellent effect of improving the efficiency of electrical stimulation to the vagus nerve by stimulating the brain with two types of stimulation by adding acoustic stimulation together when stimulating the vagus nerve through the electrodes in contact with the ears.

## Description

### Technical Field

The present disclosure relates to a method for controlling a complex stimulation that adds several stimuli simultaneously. More specifically, the present disclosure relates to a method for controlling a complex stimulation that adds an electrical stimulus to the vagus nerve and an auditory stimulus to the ear together.

### Background Art

The vagus nerve, which is one of the cranial nerves, corresponds to the tenth cranial nerve. The vagus nerve originates from the brain and is distributed throughout the face, chest, and abdomen, and is a mixed nerve containing parasympathetic nerve fibers and is involved in the regulation of the parasympathetic nerves that act on the heart, lungs, and digestive tract. The vagus nerve has the longest and most complex structure among twelve pairs of cranial nerves, and includes both sensory nerve fibers and motor nerve fibers.

It is known that vagus nerve stimulation devices for the treatment of epilepsy and depression have been approved by the U.S. Food and Drug Administration (FDA), and target the cervical branch located in the neck for stimulation. However, this electrical stimulation of the cervical branch of the vagus nerve involves making a direct incision in the skin, exposing the cervical branch of the vagus nerve, wrapping an electric coil around the outside thereof, and mounting a microchip thereto, so the electrical stimulation cannot be used in the area of self-treatment by the general public.

A vagus nerve stimulation device for the treatment of epilepsy and depression affects the brain by controlling nerve signals entering the brain, and is a device based on neuromodulation. Neuromodulation is a physiological process in which a given neuron uses one or more chemicals to modulate a diverse population of neurons, and differs from synaptic transmission in which neurotransmitters are released from axon terminals by targeting fast-acting receptors on one specific partner neuron. A method of applying electrical or chemical stimuli to the nervous system is being studied as a method to improve a nervous system function or reduce chronic pain by using this neuromodulation. The vagus nerve stimulation device and deep brain stimulation device described earlier have very limited applications since the devices that apply electrical stimuli to the nerves are directly attached to the nerves inside the human body.

Methods that directly affect the brain from outside the human body include a magnetic stimulation method, in which a magnetic field applied from outside the brain activates the brain, and a transcranial electrical stimulation method, in which electricity flowing through an electrode attached to the transcranial cranium activates the cerebral cortex. A transcranial magnetic stimulation method and a transcranial electrical stimulation method, which are non-invasive methods, have the effect of directly influencing the brain, but have limitation in that the area of the brain stimulated is limited to an area near the skull.

### Disclosure

### Technical Problem

The present disclosure is intended to solve the problems of the prior art described above, and is intended to provide a control method that is able to improve the effect of vagus nerve stimulation by adding acoustic stimuli that affect the brain when controlling electrical stimuli that stimulate the vagus nerve.

### Technical Solution

In order to accomplish the above objective, a method for controlling a complex stimulation according to an aspect of the present disclosure, which is a method for controlling a complex stimulation device having electrical stimulation units comprising electrodes in contact with human ears and sound output units for stimulating hearing by using sound waves, wherein the sound output units generate sound waves having different frequencies in both ears, and control a frequency difference between the sound waves applied to the both ears on a basis of frequencies of electrical stimuli applied to the electrodes.

When an electrical stimulus having an equal frequency is applied to each of both ears, or when an electrical stimulus is applied to only one of the both ears, the sound waves applied to the both ears may be controlled so that the frequency of the electrical stimulus applied to each of the electrodes is equal to a frequency difference between the sound waves applied to the both ears.

When electrical stimuli having different frequencies are applied to the electrodes of both ears, respectively, the sound waves applied to the both ears may be controlled so that a frequency difference between the electrical stimuli applied to the electrodes in contact with the vagus nerves of the both ears is equal to a frequency difference between the sound waves applied to the both ears.

In this case, the frequency difference between the electrical stimuli applied to the electrodes in contact with both ears may be 5Hz or more.

The frequencies of the electrical stimuli applied to the electrodes may be in a range of 10Hz to 120Hz.

Intensities of the electrical stimuli applied to the electrodes may be in a range of 3V to 80V.

Pulse widths of the electrical stimuli applied to the electrodes may be in a range of 10µs to 250µs.

A complex stimulation device according to another aspect of the present disclosure includes: two electrodes in contact with the vagus nerves, respectively, located in both ears; two sound output units that stimulate hearing in the both ears, respectively; and a control unit that controls electrical stimuli applied to the electrodes and sound waves output from the sound output units, wherein the control unit controls the sound output units to generate sound waves having different frequencies, respectively, and controls a frequency difference between sound waves applied to the both ears on a basis of frequencies of the electrical stimuli applied to the electrodes.

When the control unit controls an electrical stimulus having an equal frequency to be applied to each of both ears, or when the control unit controls an electrical stimulus to be applied to only one of the both ears, the control unit may control the sound waves applied to the both ears so that the frequency of the electrical stimulus applied to each of the electrodes is equal to a frequency difference between the sound waves applied to the both ears.

When the control unit controls electrical stimuli having different frequencies to be applied to the electrodes of both ears, respectively, the control unit may control the sound waves applied to the both ears so that a frequency difference between the electrical stimuli applied to the electrodes in contact with the vagus nerves of the both ears is equal to a frequency difference between the sound waves applied to the both ears.

### Advantageous Effects

The complex stimulation device of the present disclosure having the configuration as described above has an excellent effect of improving the efficiency of electrical stimuli to the vagus nerve by stimulating the brain with two types of stimuli by adding acoustic stimuli when stimulating the vagus nerve through the electrodes in contact with the ears.

### Description of Drawings

FIG. 1 is a diagram illustrating elements controlling electrical stimuli applied to the vagus nerve.
FIG. 2 is a diagram illustrating the configuration of a vagus nerve stimulation device according to an embodiment of the present disclosure.

### <Description of the Reference Numerals in the Drawings>

| | | | |
|---|---|---|---|
| 10: | Vagus nerve stimulation device | 100: | Left wearable unit |
| 110: | Electrode | 120: | Sound output unit |
| 200: | Right wearable unit | 210: | Electrode |
| 220: | Sound output unit | 300: | Control unit |

### Mode for Invention

An embodiment according to the present disclosure will be described in detail with reference to the attached drawings.

However, the embodiment of the present disclosure may be modified in several different ways, and the scope of the present disclosure is not limited to the embodiment described below. The shapes and sizes of elements in the drawings may be exaggerated for clearer explanation, and elements indicated by the same symbols in the drawings are the same elements.

In addition, throughout the specification, when a part is described to be "connected" to another part, this includes not only a case in which the part is "directly connected" to the another part but also a case in which the part is "electrically connected" to the another part with another element therebetween. In addition, when a part is described to "include" or "have" a certain component, this means that the part does not exclude other components but may further include or be provided with other components, unless specifically stated to the contrary.

In addition, terms such as "first" and "second" are used to distinguish one component from another component, and the scope of the claims should not be limited by these terms. For example, a first component may be named a second component, and similarly, the second component may also be named a first component.

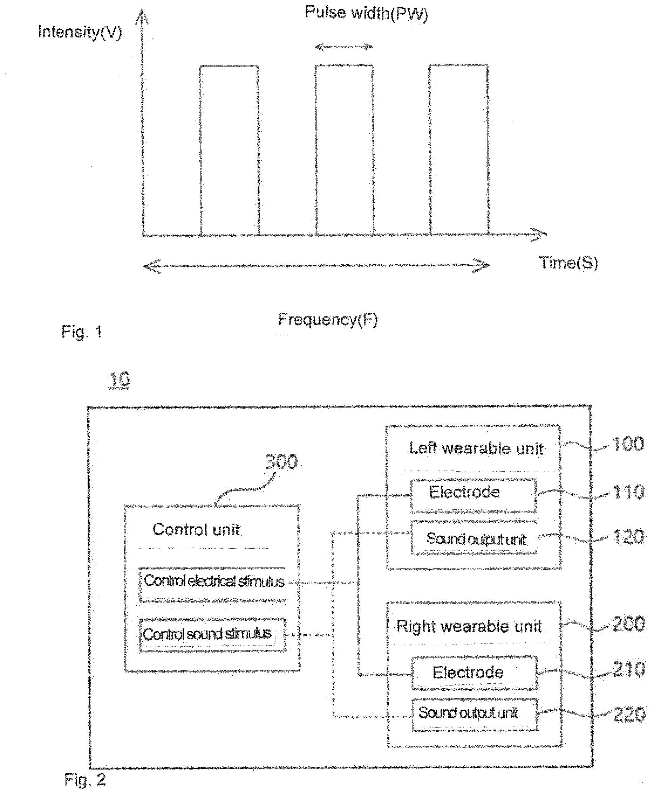

FIG. 1 is a diagram illustrating elements controlling an electrical stimuli applied to the vagus nerve.

Electrical stimuli to stimulate the vagus nerve are controlled in the form of pulse waves, and the intensity V, pulse width PW, and frequency F thereof may be controlled. For an electrical stimulus, the general term and unit of frequency, Hz, is used only for an alternating current, and the term and unit of pulse frequency, pps (pulses per second), is used separately for pulsed current, but a frequency and Hz may also be used for pulse waves, and are used in this specification.

In stimulation treatment using electrical stimuli, it is common to perform stimulation treatment with various types of treatment waveforms by controlling an intensity V, a pulse width PW, and a frequency F. This control of electrical stimuli is to control the intensity of stimuli felt by a user so that the intensity is not too strong or too weak, and is also intended to increase the efficiency of stimulation through changes rather than continuously applying a constant stimulation.

In developing a neuromodulation technology to activate a brain function, the inventor of the present disclosure developed a neuromodulation technology using the vagus nerve, unlike a transcranial magnetic stimulation method or transcranial electrical stimulation method, and particularly, developed a technology to activate the limbic system and cerebral cortex via multiple central nerve nuclei by non-invasively stimulating the vagus nerve of the outer ear to stimulate afferent sensory nerves.

The technology of transcranial magnetic stimulation or transcranial electrical stimulation has the characteristic of stimulating the cerebral cortex from the outside, but vagus nerve stimulation according to the present disclosure is a technology capable of effectively stimulating the brainstem and cerebrum by stimulating the deep part of the brain via the vagus nerve, the tenth cranial nerve.

Meanwhile, the activity of the cerebral cortex is able to be controlled through acoustic stimuli, and this effect is able to promote changes by affecting neural synapses related to degenerative brain diseases on the basis of neural plasticity. The non-invasive vagus nerve stimulation of the present disclosure is able to improve cerebral neural plasticity by stimulating the cerebral cortex through the nucleus tractus solitarius, locus coeruleus, and nucleus basalis, thereby improving learning ability by promoting the formation of new synapses. Changes in cerebral synapses caused by acoustic stimuli are able to improve treatment effectiveness by causing synergy with the improvement of cerebral plasticity caused by the non-invasive vagus nerve stimulation of the present disclosure.

In particular, the inventor of the present disclosure found that when an acoustic stimulus is applied to the auditory nerve along with an electrical stimulus to the vagus nerve of the ear, brain activation progresses actively under some conditions. Afterwards, in order to find specific conditions that induce brain activation, various conditions were experimented and it was found that there was a relationship between the frequency of an electrical stimulus and the frequency of an acoustic stimulus.

In this case, in the control range of electrical stimuli that may be applied to the vagus nerve of the ear, an intensity V is in the range of 3V to 80V, a frequency F is in the range of 10Hz to 120Hz, and a pulse width PW is in the range of 10µs to 250µs. The control range of electrical stimuli is determined by considering the range of electrical stimuli that may be applied to human nerves and the range that may be handled by non-experts.

On the basis of the frequencies of the electrical stimuli in the above range, through various studies to increase the effect of vagus nerve stimulation, the inventors of the present disclosure configured the frequencies of acoustic stimuli applied to the left and right ears to be different, and derived a method of linking a frequency difference between the acoustic stimuli to the frequency of an electrical stimulus applied to the vagus nerve of the ear.

It is widely known that the vagus nerve is a pathway that transmits nerve signals between various organs of the human body and the brain, wherein an electrical stimulus on the vagus nerve is transmitted to the brain along nerve fibers, and the electrical stimulus on the vagus nerve affects the extensive network of the brain. In this case, when the frequency of the electrical stimulus applied to the vagus nerve of the ear and the frequency of the acoustic signal applied to the auditory nerve are adjusted, the effect of the electrical stimulus on the brain and the effect of the acoustic signal on the brain interact, and thus the effect of the electrical stimulus applied to the vagus nerve is believed to be improved.

The frequencies of the acoustic stimuli applied to the left and right ears are configured to be different, and there are two specific methods for linking a frequency difference between the acoustic stimuli to the frequency of an electrical stimulus applied to the vagus nerve of the ear.

First, a first method is a method suitable when an electrical stimulus having an equal frequency is applied to each of both ears or when an electrical stimulus is applied to only one of both ears. In this case, applying the equal electrical stimuli to both ears does not mean that the stimuli are physically completely identical, and is a concept that includes some errors depending on the configuration of a device by applying the same conditions to two electrodes.

When the frequencies of electrical stimuli applied to the electrodes of both ears or the electrode of one ear are equal, the frequencies of acoustic stimuli applied to the left and right ears are configured to be different, and a frequency difference between the acoustic stimuli applied to the both ears is controlled to be equal to the frequency of each of the electrical stimuli, thereby improving the effect of electrical stimuli on the vagus nerve.

For example, when each of electrical stimuli applied to both ears or one ear is 20Hz, a frequency difference between acoustic stimuli applied to the left and right ears is controlled to be 20Hz. In this case, the frequency range of the acoustic stimuli is not particularly limited as long as the frequency difference between the acoustic stimuli is equal to the frequency of the electrical stimulus, and a preferred acoustic frequency range may be applied by reflecting technical details on acoustic stimuli that have been studied so far.

Next, a second method is a method suitable when the frequencies of electrical stimuli applied to both ears are different.

In stimulation treatment using electrical stimuli, it is common to perform the stimulation treatment with various types of treatment waveforms by controlling an intensity V, a pulse width PW, and a frequency F. This control of electrical stimuli is to control the intensity of a stimulus felt by a user so that the intensity is not too strong or too weak, and is also intended to increase the efficiency of stimulation through changes rather than continuously applying a constant stimulation.

The inventor of the present disclosure studied an effect on the brain caused by neuromodulation when applying electrical stimuli to the vagus nerve of the ear, and developed a method of activating the brain by indirectly stimulating the brain, unlike a transcranial magnetic stimulation method or a transcranial electrical stimulation method. As one of the methods, in the process of stimulating the vagus nerve of both ears, it was found that when electrical stimuli having different frequencies are applied to the both ears, brain activation occurs actively. Furthermore, it was found that the effect of electrical stimuli on the vagus nerve was improved by configuring the frequencies of acoustic stimuli applied to the left and right ears to be different, and controlling a frequency difference between the acoustic stimuli applied to the both ears to be equal to a frequency difference between the electrical stimuli. In this case, when a frequency difference between electrical stimuli applied to both ears is 5Hz or more, the effect of inducing brain activation due to the frequency difference between electrical stimuli appears.

For example, when the frequencies of electrical stimuli applied to the left and right ears are 30Hz and 40Hz, respectively, a frequency difference between acoustic stimuli applied to the left and right ears is controlled to be 10Hz. As described above, the frequency range of the acoustic stimuli is not particularly limited as long as the frequency difference between the acoustic stimuli is equal to a frequency difference between the electrical stimuli, and a preferred acoustic frequency range may be applied by reflecting technical details on acoustic stimuli that have been studied so far.

FIG. 2 is a diagram illustrating the configuration of a vagus nerve stimulation device according to an embodiment of the present disclosure.

The vagus nerve stimulation device 10 of the present disclosure includes a left wearable unit 100 worn on the left ear of a user and a right wearable unit 200 worn on the right ear of a user, wherein the electrodes 110 and 210 for applying electrical stimuli to the vagus nerve of the ear and sound output units 120 and 220 are formed in the left wearable unit 100 and the right wearable unit 200, respectively.

The specific structures of the left wearable unit 100 and the right wearable unit 200 may be applied in various ways without harming the characteristics of the present disclosure. The structure of the left wearable unit 100 and the right wearable unit 200 may be a structure in which the left wearable unit 100 and the right wearable unit 200 are separated from each other and worn separately, and in this case, the structure for fixing the wearable units to the ear of a user is not limited. For example, each of the structures of the left wearable unit and the right wearable unit may be fixed to the ear by a structure inserted into the ear canal, or may be fixed to the ear by a separate fixing means such as an ear hook, and the shape of the fixing means may also be applied without limitation as long as the shape does not impair the characteristics of the present disclosure. Furthermore, the stimulation device may further include a connection part that structurally connects the left wearable unit 100 and the right wearable unit 200 to each other, wherein any conventional form of the structure of the connection part may be applied without limitation, such as when the connection part is located on the head or on the back of the neck. Even when including the connection part, the left wearable unit 100 and the right wearable unit 200 may be fixed to the ear of a wearer by the elasticity of the connection part, or may be fixed to the ear by a separate fixing means.

The specific structure of each of the electrodes 110 and 210 formed in the left wearable unit 100 and the right wearable unit 200, respectively, may also be applied in various ways without impairing the characteristics of the present disclosure. In particular, existing technologies may be applied in various ways to the location and shape of the electrode in contact with the ear to stimulate the vagus nerve, and the electrode may typically be formed to be in contact with the cymba concha of the ear, or may be formed to be inserted into and contact the external auditory canal.

The specific structure of each of the sound output units 120 and 220 formed in the left wearable unit 100 and the right wearable unit 200, respectively, may also be applied in various ways without impairing the characteristics of the present disclosure, and technologies used in headphones and earphones may be applied. When each of the electrodes 110 and 210 is inserted into the external auditory canal, it is preferable to form a hole through which sound can be transmitted to the inside of each of the electrodes 110 and 210.

A control unit 300 is configured to control an electrical stimulus applied to the vagus nerve through the electrodes 110 and 210 formed in the left wearable unit 100 and the right wearable unit 200, respectively, and an acoustic stimulus output from each of the sound output units 120 and 220. In this case, the control unit may control electrical stimuli applied to the electrode 110 of the left wearable unit 100 and the electrode 210 of the right wearable unit 200 together or individually. The control unit 300 can control the intensity V, pulse width PW, and frequency F of an electrical stimulus. In the control range of an electrical stimulus controlled by the control unit 300, the intensity V is in the range of 3V to 80V, the frequency F is in the range of 10Hz to 120Hz, and the pulse width PW is in the range of 10µs to 250µs. In addition, the control unit 300 may control the frequencies of an acoustic stimulus output from the sound output unit 120 of the left wearable unit 100 and an acoustic stimulus output from the sound output unit 220 of the right wearable unit 200 to be different from each other. Specifically, the control unit 300 may control a frequency difference between acoustic stimuli applied to the left and right ears on the basis of the frequencies of electrical stimuli applied to the electrodes 110 and 210. Specifically, when an electrical stimulus having equal frequency is applied to each of the electrodes 110 and 210 formed in the left wearable unit 100 and the right wearable unit 200, respectively, the control unit 300 may control a frequency difference between an acoustic stimulus output from the sound output unit 120 of the left wearable unit 100 and an acoustic stimulus output from the sound output unit 220 of the right wearable unit 200 to be equal to the frequency of the electrical stimulus. In addition, when an electrical stimulus is applied to only one of the electrodes 110 and 210 formed on the left wearable unit 100 and the right wearable unit 200, respectively, the control unit 300 may control a frequency difference between an acoustic stimulus output from the sound output unit 120 of the left wearable unit 100 and an acoustic stimulus output from the sound output unit 220 of the right wearable unit 200 to be equal to the frequency of the electrical stimulus. Furthermore, when electrical stimuli having different frequencies are applied to the electrodes 110 and 210 formed on the left wearable unit 100 and the right wearable unit 200, respectively, the control unit 300 may control a frequency difference between an acoustic stimulus output from the sound output unit 120 of the left wearable unit 100 and an acoustic stimulus output from the sound output unit 220 of the right wearable unit 200 to be equal to a frequency difference between the electrical stimuli.

When the control unit 300 controls the frequencies F of electrical stimuli applied to the vagus nerves of both ears to be different, the control unit 300 may control the difference of the frequencies to be 5Hz or more.

The present disclosure has been described above through the exemplary embodiment, but the above-described embodiment is merely illustrative explanation of the technical idea of the present disclosure, and those skilled in the art will understand that the embodiment may be variously changed without departing from the technical idea of the present disclosure. Those skilled in the art will understand. Therefore, the scope of protection of the present disclosure should be interpreted on the basis of the matters stated in the patent claims, not a specific embodiment, and all technical ideas within the equivalent scope should be interpreted as being included in the scope of the claims of the present disclosure.

## Claims

1. A method for controlling a complex stimulation, which is a method for controlling a complex stimulation device having electrical stimulation units comprising electrodes in contact with human ears and sound output units for stimulating hearing by using sound waves, wherein the sound output units generate sound waves having different frequencies in both ears, and control a frequency difference between the sound waves applied to the both ears on a basis of frequencies of electrical stimuli applied to the electrodes.

2. The method of claim 1, wherein when an electrical stimulus having an equal frequency is applied to each of both ears, or when an electrical stimulus is applied to only one of the both ears, the sound waves applied to the both ears are controlled so that the frequency of the electrical stimulus applied to each of the electrodes is equal to a frequency difference between the sound waves applied to the both ears.

3. The method of claim 1, wherein when electrical stimuli having different frequencies are applied to the electrodes of both ears, respectively, the sound waves applied to the both ears are controlled so that a frequency difference between the electrical stimuli applied to the electrodes in contact with the vagus nerves of the both ears is equal to a frequency difference between the sound waves applied to the both ears.

4. The method of claim 3, wherein the frequency difference between the electrical stimuli applied to the electrodes in contact with both ears is 5Hz or more.

5. The method of claim 1, wherein the frequencies of the electrical stimuli applied to the electrodes are in a range of 10Hz to 120Hz.

6. The method of claim 1, wherein intensities of the electrical stimuli applied to the electrodes are in a range of 3V to 80V.

7. The method of claim 1, wherein pulse widths of the electrical stimuli applied to the electrodes are in a range of lOps to 250µs.

8. A complex stimulation device comprising:
two electrodes in contact with the vagus nerves, respectively, located in both ears;
two sound output units that stimulate hearing in the both ears, respectively; and
a control unit that controls electrical stimuli applied to the electrodes and sound waves output from the sound output units,
wherein the control unit controls the sound output units to generate sound waves having different frequencies, respectively, and controls a frequency difference between sound waves applied to the both ears on a basis of frequencies of the electrical stimuli applied to the electrodes.

9. The complex stimulation device of claim 8, wherein when the control unit controls an electrical stimulus having an equal frequency to be applied to each of both ears, or when the control unit controls an electrical stimulus to be applied to only one of the both ears, the control unit controls the sound waves applied to the both ears so that the frequency of the electrical stimulus applied to each of the electrodes is equal to a frequency difference between the sound waves applied to the both ears.

10. The complex stimulation device of claim 8, wherein when the control unit controls electrical stimuli having different frequencies to be applied to the electrodes of both ears, respectively, the control unit controls the sound waves applied to the both ears so that a frequency difference between the electrical stimuli applied to the electrodes in contact with the vagus nerves of the both ears is equal to a frequency difference between the sound waves applied to the both ears.
